(19) Europäisches Patentamt
European Patent Office
Office européen des brevets

(11) Publication number: 0 215 171 B1

(12) EUROPEAN PATENT SPECIFICATION

(45) Date of publication of patent specification: 22.11.90

(51) Int. Cl.[5]: C 07 K 7/06, A 61 K 37/02, C 07 K 7/26

(21) Application number: 85308770.8

(22) Date of filing: 02.12.85

# (54) Peptides.

(30) Priority: 12.09.85 US 775488

(43) Date of publication of application: 25.03.87 Bulletin 87/13

(45) Publication of the grant of the patent: 22.11.90 Bulletin 90/47

(84) Designated Contracting States: AT BE CH DE FR GB IT LI LU NL SE

(56) References cited:
EP-A- 192 742
EP-A-0 029 579
EP-A-0 203 031
WO-A-86/01516
GB-A-2 095 261
US-A-4 282 143
US-A-4 328 135

Chemical Abstracts, vol.102, 1985, page82, no. 160733u, Columbus Ohio, US; L TORRES-ALEMAN et al.: "Inhibition of growth of a prelactin and growth hormone secreting pituitary tumor in rats by d-tryptophan-6 analog of lutelrising hormone-releasing hormone" & PROC. NATL. ACAD. SCI. USA, 1985, 82(4), 1252-6

(73) Proprietor: ADMINISTRATORS OF THE TULANE EDUCATIONAL FUND
1430 Tulane Avenue Tulane University
New Orleans Louisiana 70112 (US)

(72) Inventor: Coy, David H.
4319 Perrier Street
New Orleans Louisiana 70115 (US)
Inventor: Murphy, William A.
Route 8, Box 979
Covington Louisiana 70433 (US)
Inventor: Heiman, Mark L.
2513 Jasmine Street
New Orleans Louisiana 70112 (US)

(74) Representative: Deans, Michael John Percy et al
Lloyd Wise, Tregear & CO. Norman House
105-109 Strand
London WC2R 0AE(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 215 171 B1

(56) References cited:

Chemical Abstracts, vol.104, 1986, page 71, no 28941x, Columbus, Ohio, US; W A MURPHY et al.: "Octapeptide analogs of somatostatin exhibiting greatly enhanced in vivo and in vitro inhibition of growth hormone secretion in the rat & BIOCHEM: BIOPHYS RES COMMUN 1985, 132(3), 922 8

Chemical Abstracts, vol.107, 1987, page 79, bo. 147517k, Columbus, Ohio, US; W A MURPHY et al.: "Inhibition of rat prostate tumor growth by an octapeptide analog somatostatin" & LIFE SCI. 1987, 40(26), 2515-22 (Public. of the invention)

**Description**

This invention relates to peptides.

A number of somatostatin analogs exhibiting GH-release-inhibiting activity have been described in the literature, including analogs containing fewer than the naturally occurring fourteen amino acids. For example, Coy et al. US Patent No. 4,485,101, the disclosure of which is hereby incorporated by reference, describes dodecapeptides having an N-terminal acetyl group, a C-terminal $NH_2$, D-Trp at position 6, and p-Cl-Phe at position 4. (herein, when no designation of configuration is given, the L-isomer is intended).

The present invention features an octapeptide of the formula:

D-β-naphthylalanine-Cys-Tyr-D-Trp-Lys-Val-Cys-Thr-$NH_2$.

In preferred embodiments, a therapeutically effective amount of the therapeutic compound and a pharmaceutically acceptable carrier substance, e.g. magnesium carbonate, lactose, or a phospholipid with which the therapeutic compound can form a micelle, together form a therapeutic composition, e.g. a pill, tablet, capsule, or liquid for oral administration to a human patient, a spreadable cream, gel, lotion, or ointment for application to the skin of a human patient in need of the compound, a liquid capable of being administered nasally as drops or spray, or a liquid capable of intravenous, parenteral, subcutaneous, or intraperitoneal administration. The pill, tablet or capsule can be coated with a substance capable of protecting the composition from the gastric acid in the patient's stomach for a period of time sufficient to allow the composition to pass undisintegrated into the patient's small intestine. The therapeutic composition can also be in the form of a biodegradable sustained release formulation for intramuscular administration.

The compound of the invention is active in inhibiting the secretion of GH, insulin, and glucagon. Further, the aromatic lipophilic N-terminal end can provide long-lasting *in vivo* activity.

Other features and advantages of the invention will be apparent from the following description of the preferred embodiments thereof.

The compound of the invention has the formula recited above, and is an octapeptide analog of somatostatin, having D-Trp at position 4; D-β-naphthylalanine at position 1; Tyr at position 3; and Val at position 6.

The compound can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

The first step in the synthesis of the compound of the invention was the synthesis of the intermediate tert-butyloxycarbonyl - D - β - naphthylalanic - S - methylbenzyl - Cys - Tyr - D - Trp - $N^{\varepsilon}$ - benzyloxycarbonyl - Lys - Val - S - methylbenzyl - Cys - O - benzyl - Thr - benzyhydrylaminic resin, as follows:

Benzhydrylamine-polystyrene resin (Vega Biochemicals, Inc.) (1.30 g, 0.5 mmole) in the chloride ion form was placed in the reaction vessel of a Beckman 990B peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; (f) 10% triethylamine in chloroform.

The neutralized resin was stirred with Boc-O-benzyl-threonine and diisopropylcarbodiimide (1.5 mmole each) in methylene chloride for 1 h and the resulting amino acid resin was then cycled through steps (a) to (g) in the above wash program. The following amino acids (1.5 mmole) were then coupled successively by the same procedure: Boc-S-methylbenzyl-Cys, Boc-Val, Boc-Ne-benzyloxycarbonyllysine, Boc-D-Trp, Boc-Tyr, Boc-S-methylbenzyl-Cys, Boc-D-β-naphylalanine. After washing and drying, the completed resin weighed 1.89 g.

The resin (1.89 g, 0.5 mmole) was then mixed with anisole (4 ml) and anhydrous hydrogen fluoride (36 ml) at 0°C and stirred for 45 min. Excess hydrogen fluoride was evaporated rapidly under a stream of dry nitrogen and free peptide precipitated and washed with ether. The crude peptide was then dissolved in 800 ml of 90% acetic acid to which was added $I_2$ in methanol until a permanent brown color was present. The solution was then stirred for 1 h before removing the solvent *in vacuo*. The resulting oil was dissolved in a minimum volume of 50% acetic acid and eluted on a column (2.5 × 100 mm) of Sephadex G—25. Fractions containing a major component by uv absorption and thin layer chromatography were then pooled, evaporated to a small volume, and applied to a column (2.5 × 50 cm) of Whatman LRP—1 octadecylsilane (15—20 uM).

The column was eluted with a linear gradient of 10—50% acetonitrile in 0.1% trifluoroacetic acid in water. Fractions were examined by thin layer chromatography and analytical high performance liquid chromatography and pooled to give maximum purity. Repeated lyophilization of the solution from water gave 138 mg of the product as a white, fluffy powder.

The product was found to be homogeneous by Hplc and Tlc. Amino acid analysis of an acid hydrolysate confirmed the composition of the octapeptide.

The resin, which weighed 1.78 g, was then subjected to hydrogen fluoride cleavage and $I_2$ cyclization,

as described above, to produce 170 mg of lyophilized octapeptide, which was found to be homogeneous by Hplc and Tlc. Amino acid analysis of an acid hydrolysate confirmed the composition of the octapeptide.

The compound of the invention was prepared from tert-butyloxycarbonyl - D - β - naphthylalanic - S - methylbenzyl - Cys - Tyr - D - Trp - $N^{\varepsilon}$ - benzyloxycarbonyl - Lys - Val - S - methylbenzyl - Cys - O - Benzyl - Thr - benzhydrylaminic resin according to conventional techniques.

When administered to mammals (e.g. orally, topically, intravenously, parenterally in a sustained release, biodegradable form, nasally, or by suppository), the compound can be effective to inhibit GH release as well as to inhibit insulin, glucagon, and pancreatic exocrine secretion, and to therapeutically affect the central nervous system.

The compound can be administered to a mammal, e.g. a human, in the dosages used for somatostatin or, because of its greater potency, in smaller dosages. The compound of the invention can be used for the treatment of cancer, particularly growth hormone-dependent cancer (e.g., bone, cartilage, pancreas (endocrine and exocrine), prostate, or breast), acromegaly and related hypersecretory endocrine states, or of bleeding ulcers in emergency patients and in those suffering from pancreatitis or diarrhea. The compound can also be used in the management of diabetes and to protect the liver of patients suffering from cirrhosis or hepatitis. The compound can also be used to treat Alzheimer's disease, as an analgesic to treat pain by acting specifically on certain opiate receptors, and as a gastric cytoprotective compound ulcer therapy. The compound can also be used to treat certain types of mushroom poisoning.

The compound can also be used to treat diabetes-related retinopathy. The anti-cancer activity of the compound may be related to its ability to antagonize cancer-related growth factors such as epidermal growth factor.

The compound can be administered to a mammal, e.g., a human, in a dosage of 0.01 to 1000 mcg/kg/day, preferably 0.1 to 100 mcg/kg/day.

## Claims

1. An octapeptide of the formula

$$D\text{-}\beta\text{-naphthylalanine-Cys-Tyr-D-Trp-Lys-Val-Cys-Thr-}NH_2,$$

or a pharmaceutically acceptable salt thereof.

2. A therapeutic composition comprising a therapeutically effective amount of an octapeptide according to Claim 1 together with a pharmaceutically acceptable carrier substance.

3. A therapeutic composition according to Claim 2, wherein said composition is in the form of a pill, tablet, or capsule for oral administration, optionally coated with a substance capable of protecting said composition from the gastric acid in the stomach of a patient for a period of time sufficient to allow said composition to pass undistintegrated into the small intestine of said patient; in the form of a liquid for oral administration; in the form of a cream, gel, spray, or ointment for application to the skin; in the form of a liquid capable of being administered nasally as drops or spray; in the form of a liquid for intravenous, subcutaneous, parenteral, or intraperitoneal administration; or in the form of a biodegradable sustained release composition for intramuscular administration.

4. An octapeptide according to Claim 1 for use in therapy in inhibiting the release of or causing a reduction in growth hormone, insulin, glucagon, or pancreatic exocrine secretion, in the treatment of cancer, in the treatment of bleeding ulcers, in the management of diabetes, in the protection of the liver in patients suffering from cirrhosis or hepatitis, in Alzheimer's disease, as an analgesic, in ulcer therapy, in mushroom poisoning, or in diabetes-related retinopathy.

5. A method of synthesizing an octopeptide of the formula:

$$D\text{-}\beta\text{-naphthylalanine-Cys-Tyr-D-Trp-Lys-Val-Cys-Thr-}NH_2$$

comprising the steps of: forming a blocked amino acid resin of threonine; coupling successively to said threonine resin the blocked amino acids cysteine, valine, lysine, D-tryptophan tyrosine, cysteine, and tert-butyloxycarbonyl-D-β-naphthylalanine; washing and drying the product of said coupling; treating said washed and dried product to remove said blocking groups; and purifying said unblocked product.

## Patentansprüche

1. Octapeptid der Formel

$$D\text{-}\beta\text{-Naphthylalanin-Cys-Tyr-D-Trp-Lys-Val-Cys-Thr-}NH_2$$

oder ein pharmazeutisch verträgliches Salz desselben.

2. Therapeutische Zusammensetzung, die eine therapeutisch wirksame Menge eines Octapeptides nach Anspruch 1 zusammen mit einer pharmazeutisch verträglichen Trägersubstanz enthält.

3. Therapeutische Zusammensetzung nach Anspruch 2, worin die genannte Zusammensetzung die

Form einer Pille, Tablette, oder Kapsel für die perorale Verabreichung aufweist, die gegebenenfalls mit einer Substanz beschichtet ist, die dazu geeignet ist, die genannte Zusammensetzung vor der Magensäure im Magen eines Patienten für einen Zeitraum zu schützen, der ausreicht, um zuzulassen, daß die genannte Zusammensetzung unzersetzt in den Dünndarm des genannten Patienten gelangt; die Form einer Flüssigkeit für die perorale Verabreichung besitzt; die Form einer Creme, eines Gels, Sprays oder einer Salbe für die Anwendung auf die Haut aufweist; die Form einer Flüssigkeit besitzt, die dazu geeignet ist, nasal als Tropfen oder Spray verabreicht zu werden; die Form einer Flüssigkeit für die intravenöse, subkutane, parenterale oder intraperitoneale Verabreichung aufweist; oder die Form einer biologisch abbaubaren Zusammensetzung mit lang anhaltender Freisetzung für die intramuskuläre Verabreichung besitzt.

4. Octapeptid nach Anspruch 1 für die Verwendung in der Therapie zur Hemmung der Freisetzung oder zur Verringerung der Ausscheidung von Wachtsumshormon, Insulin, Glucagon oder Pankreasexocrin bei der Behandlung von Krebs, blutenden Geschwüren, Diabetes, dem Schutz der Leber von Patienten, die an Cirrhose oder Hepatitis leiden, der Alzheimerschen Krankheit, als Analgetikum, bei der Ulkustherapie, bei Pilzvergiftungen oder mit Diabetes einhergehender Retinopathie.

5. Verfahren zur Herstellung eines Octapeptids der Formel

D-β-Naphthylalanin-Cys-Tyr-D-Trp-Lys-Val-Cys-Thr-$NH_2$

umfassend die Schritte: Bilden eines geblockten Aminosäureharzes von Threonin; aufeinanderfolgenes Koppeln der geblockten Aminosäuren Cystein, Valin, Lysin, D-Tryptophan, Tyrosin, Cystein und tert.-Butyloxycarbonyl-D-β-Naphthylalanin mit dem genannten Threoninharz; Waschen und Trocknen des genannten Kopplungsproduktes; Behandlung des genannten, gewaschenen und getrockneten Produktes, um die genannten Blockierungsgruppen zu entfernen; und Reinigen des genannten entblockten Produktes.

**Revendications**

1. Un octapeptide de formule

D-β-naphthylalanine-Cys-Tyr-D-Trp-Lys-Val-Cys-Thr-$NH_2$,

ou un de ses sels pharmaceutiquement acceptables.

2. Une composition thérapeutique comprenant une quantité thérapeutiquement suffisante d'un octapeptide selon la revendication 1 associé à un excipient pharmaceutiquement acceptable.

3. Une composition thérapeutique selon la revendication 2, ladite composition étant sous forme de comprimé, pillule ou capsule lorsqu'il s'agit d'une administration par voie orale, éventuellement enrobée d'une substance capable de protéger cette composition des acides gastriques de l'estomac d'un patient suffisamment longtemps pour lui permettre de transiter sans désagrégation jusqu'à l'intestin grêle du patient; sous forme d'un liquide pour une administration par voie orale; sous forme de crème, de gel, de pulvérisation ou de pommade pour une application sur la peau; sous forme de liquide pouvant être administré par voie nasale (gouttes ou pulvérisation); sous forme de liquide pour injection par voies intraveineuse, sous-cutanée, parentérale ou intrapéritonéale; ou sous forme d'une composition biodégradable à libération retardée pour une administration intramusculaire.

4. Un octapeptide selon la revendication 1 pour une utilisation thérapeutique dans le traitement du cancer, dans le traitement des ulcères saignants, dans le traitement des diabètes, dans la protection du foie chez les patients souffrant de cirrhose ou d'hépatite, dans la maladie d'Alzheimer, comme analgésique, dans le traitement des ulcères, des empoisonnements dûs aux champignons, des diabètes dits rétinopathiques, soit en inhibant la libération de l'hormone de croissance ou soit en causant une réduction de l'hormone de croissance, insuline, glucacon, ou la sécrétion d'exocrine pancréatique.

5. Une méthode de synthèse d'un octapeptide de formule:

D-β-naphthylalanine-Cys-Tyr-D-Trp-Lys-Val-Cys-Thr-$NH_2$

comprenant les étapes suivantes: formation d'une résine d'acide aminé bloqué avec la thréonine, couplages successifs des acides aminés bloqués cystéine, valine, lysine, D-tryptophan tyrosine, cystéine et tert-butyloxycarbonyl-D-β-naphtylalanine avec la résine à la thréonine, précédemment obtenue, lavage et séchage du produit obtenu lors de l'étape de couplage, traitement du produit lavé et séché pour éliminer les groupes bloquants et, purification des produits débloqués.